# EUROPEAN PATENT APPLICATION

(11) **EP 3 839 959 A1**
(43) Date of publication of application: **23.06.2021**
(21) Application number: 19217941.4
(22) Date of filing: 19.12.2019

(54) **TRANSDERMAL SEDATIVE RELEASE CONTROL IN AUTONOMOUS IMAGING**

(71) Applicant: Koninklijke Philips N.V., 5656 AG Eindhoven (NL)
(72) Inventor: JOHNSON, Mark Thomas, 5656 AE Eindhoven (NL); WEISS, Steffen, 5656 AE Eindhoven (NL); VUPPALA, Sunil Kumar, 5656 AE Eindhoven (NL); SISODIA, Rajendra Singh, 5656 AE Eindhoven (NL); LEUSSLER, Christoph, 5656 AE Eindhoven (NL); VOGTMEIER, Gereon, 5656 AE Eindhoven (NL)
(74) Representative: Philips Intellectual Property & Standards

(57) **Abstract**

The present invention relates to a system and method for controlling transdermal and automatic release of a sedative during imaging of a subject. A sedation level of a patient is detected during an imaging procedure. An amount of sedative to be applied to the patient to sustain a certain level of sedation is calculated. Further, a time for releasing the amount of the sedative is calculated. The calculated amount of the sedative is released at the calculated time transdermally to the patient.

## Description

### FIELD OF THE INVENTION

The present invention relates to a system for controlling transdermal and automatic release of a sedative during imaging of a subject, and a method for controlling transdermal and automatic release of a sedative during imaging of a subject.

### BACKGROUND OF THE INVENTION

Sedative drugs are administered before or during a medical image examination when the patient feels anxious or when it is necessary to ensure that the acquired images have diagnostic quality, e.g. in pediatric examination. Moreover, some imaging approaches such as special magnetic resonance imaging sequences rely on long image acquisition times that bear the risk of patient movement resulting in decreased image quality. The probability of patient movement during the image acquisition can be decreased by increasing the dosage of sedative drugs or by giving an extra bolus before or during the scan. However, this increases the risk of complications for the patient and is economically disadvantageous. In general it is desirable only to give more sedative drug if the level of sedation is observed to have decreased below the level required to successfully complete the next scan.

A problem related to magnetic resonance imaging is the fact that the patient can only hardly be assessed from outside the scanner room, as the patient is located right in the center of the magnet bore. During autonomous imaging, particularly during autonomous imaging, it is beneficial to have a method to administer more sedative without requiring the access to the patient. Intravenous sedation administration is however very intrusive and dangerous to carry out autonomously, whilst oral sedative intake is not practical during imaging and without human action. In particular, it is desirable to repeatedly release relatively small doses of sedative to the patient whilst the patient is in the bore of the scanner.

For these reasons, it would be advantageous to have a system for applying a sedative to a patient located in a medical imaging system, that does not suffer from the above mentioned drawbacks.

### SUMMARY OF THE INVENTION

It is an object of the present invention to provide a system for controlling transdermal and automatic release of a sedative during imaging of a subject with an improved performance.

The object of the present invention is solved by the subject matter of the independent claims, wherein further embodiments are incorporated in the dependent claims.

The described embodiments similarly pertain to the system for controlling transdermal and automatic release of a sedative during imaging of a subject, and the method for controlling transdermal and automatic release of a sedative during imaging of a subject. Synergetic effects may arise from different combinations of the embodiments although they might not be described in detail.

Further on, it shall be noted that all embodiments of the present invention concerning a method, might be carried out with the order of the steps as described, nevertheless this has not to be the only and essential order of the steps of the method. The herein presented methods can be carried out with another order of the disclosed steps without departing from the respective method embodiment, unless explicitly mentioned to the contrary hereinafter.

According to a first aspect of the invention, there is provided a system for controlling transdermal and automatic release of a sedative during imaging of a subject. The system is configured for detecting a sedation level of the subject, and calculating an amount of the sedative to be released transdermally to the subject. The system is further configured for calculating a time for releasing the amount of the sedative transdermally to the subject, and releasing the calculated amount of the sedative at the calculated time transdermally to the subj ect.

The invention proposes a system for controlled transdermal release of a sedative during an imaging procedure. The imaging procedure can be at least partially automated. The sedative is applied transdermally to the patient, which results in an incorporation of the sedative through the skin in a noninvasive and easy dosed way. Further, the sedative can be applied without causing a movement of the patient, which increases the imaging quality, as the sedative can be applied while the patient may be inside of a medical scanner, and during the imaging procedure is performed. Therefore, the system is configured for detecting a sedation level of the patient in an imaging apparatus. The system calculates an amount of sedative that has to be applied to the patient in order to reach the desired sedation level. The system further calculates a time for applying the amount of sedative to the patient. The calculated amount of the sedative is further at the calculated time applied transdermally by the system to the patient. Preferably, the system can be fully automated such that it performs the steps of detecting the sedation level, calculating the amount of the sedative and the time of the application of the sedative, and applying the sedative transdermally to the patient in an automated procedure without an intervention of a user. Alternatively, the system can perform a part of the steps or steps partwise, while a user of the system can provide additional steps of the method.

In an embodiment of the invention, the system is further configured for calculating the amount of the sedative by comparing the detected sedation level with a set value of the sedation level.

The detected sedation level can be compared with a set value of sedation level necessary for this specific imaging procedure and/or this respective patient in order to receive high quality images.

In an embodiment of the invention, the system is further configured for calculating the amount of the sedative by considering variables like a type of the sedative, data of the subject, a type of an imaging procedure, and/or the purpose of sedation.

The amount of sedative necessary to obtain the set value of the sedation level and also the time of releasing the sedative can be further dependent on a type of an imaging procedure to be performed and the purpose of sedation. Further, a condition of the patient can have an influence to the desired sedation level, like for example his anxiousness, age, sex, mass, or recent measurement values of this patient. Also the type of the sedative has to be considered.

In an embodiment of the invention, the system comprises an artificial intelligence module configured for calculating the amount of the sedative by machine learning methods.

The system can comprise an artificial intelligence module, which is configured to calculate the time and the amount of the sedative to be applied. The artificial intelligence module can be trained with machine learning methods in order to determine the time and the amount of the sedative to be applied correctly. As training data for the training of the artificial intelligence module, data of a plurality of patients can be used in order to measure, respond and decide the right time to deliver an amount of the sedative. The controlled delivery can lead to some kind of standard recipes which can be linked to certain people groups. The recipe information can be double-checked with former patient data to get a reliable process with low patient risk. For evaluating this data of former patients, the artificial intelligence module can be used.

Independent variables of the artificial intelligence module used for calculating the dosage of the sedative can include the sedative type, a patient profile comprising age, gender, and/or health condition, the purpose of sedation, and a sedation level. These values can be passed through a machine-learning model in order to predict the dependent variables. Dependent variables can be the pattern of dosage and duration, or the amount and time of the application of the sedative. The patient's measured sedation level can be checked against the model outcome at regular intervals. This adaptive system can also give the feedback on suitability of the approach for a particular patient which can be used as an input for a further learning process of the artificial intelligence module.

The values of the categorical types of the independent variables can be converted to numerical values using techniques such as label encoding or frequency rate. This includes continuous monitoring and having a feedback system for patient sedation level. A specific response for a transdermal delivery can be measured and correlated with an anticipated response. Example learning models can include polynomial regression, or neural network based regression for training the model. The adaptive learning approach can be followed to make the learning process continuous and to retrain the model with latest available data. The adaptive learning is possible with feedback and monitoring methods. One way of adaptive learning is with interleaved test-then-train. Average of predicted errors can be calculated to give feedback to the system. This can help to improve the process to measure, respond and decide the right time to deliver the dosage.

In an embodiment of the invention, the system is configured for detecting the sedation level, calculating the amount of the sedative, calculating the time for releasing the amount of the sedative, and releasing the calculated amount of the sedative at the calculated time transdermally to the subject repeatedly during imaging of the subject.

The system is configured to perform the detection of the sedation level, the calculation of the time and the amount of the sedation level, and the application of the sedative consecutively and iteratively during the imaging procedure. Thus, the set value of the sedation level can be obtained reliably without the risk of applying a too high or too low dose of the sedative to the patient. With a continuous measurement of the sedation level and determination of the amount of sedative to be applied, it is possible to apply the sedative in small portions or even continuously to the patient, thus decreasing the risk of over or under dosage.

In an embodiment of the invention, calculating a time for releasing the amount of the sedative comprises determining a start time, a stop time, and a duration of releasing the sedative transdermally to the subject.

Calculating the time for releasing the amount of sedative can comprise calculating a start time, a stop time, and/or a duration of the release of the sedative. However, in combination with a known amount of sedative to be released, also rate of sedative to be released continuously over time can be calculated.

In an embodiment of the invention, the system is further configured for monitoring the amount of the sedative released transdermally to the subject.

The amount of the sedative released transdermally to the patient can be measured in this embodiment of the invention. As the uptake of the sedative by the subject may be restrained and the delivery of the sedative may be inefficient, preferably the amount of the sedative is monitored, which has entered the subject transdermally.

In an embodiment of the invention, the system is further configured for detecting a contrast agent released together with the sedative, thereby monitoring the amount of the sedative released transdermally to the subject.

A contrast agent can be released together with the sedative. The contrast agent may be incorporated by the subject in the same or in a similar extent as the sedative. Thus, a monitoring of the contrast agent can be used to monitor the amount of the sedative, that is released transdermally to the subject and that has successfully entered the body of the subject through the skin. Monitoring of the contrast agent can be performed, for example, by medical imaging systems like magnetic resonance imaging, computed tomography, positron emission tomography, or ultrasonic measurement systems.

Other approaches to assess the actual dosage could be sensors applied to the delivery devices to establish, for example, a flow from the microneedles by a flow sensor or a pressure sensor, etc. An impedance sensor or a sensor to assess the presence of a drug or a reagent in the reservoir of the iontophoretic sensor can be used. Further, a blockage of the ink jet printing head can be detected.

Furthermore, if a contrast medium has been added to the sedative to confirm or even quantitatively measure the actual delivery into the body by imaging - or indeed any other approach applied to assess the actual dosage - any deviation between the desired dosage from the model and the actual measured dosage can be compensated for.

In an embodiment of the invention, the system is further configured for releasing the amount of the sedative with one of the group consisting of: a microneedle array, iontophoresis, and high velocity jetting of the sedative.

The sedative can be released transdermally to the subject with the use of a microneedle array. A microneedle array applied to the skin of the subject can achieve precise control of, for example, a high potent sedative. A microneedle can have a diameter of less than 0.1 millimeters and a length of less than 0.1 millimeters. Thus, it may be just long enough to break through the outer layer of the skin. Preferably, hollow microneedles can be used, but also the use of solid, dissolving and/or hydro-gel forming microneedles can be possible. Specific patterns of the microneedles of the array can be provided, such that the pattern of micro-needles improves the overcoming of the barrier provided up to the dermis and/or such that the specific pattern may allow to avoid specific pain nerves towards the end of the dermis. Further, the specific pattern can be based on the anatomy of the patient where the microneedle array will be placed or it can be based on the type or modality of the scan. Further, the sedative and/or the contrast agent can be injected via a specific pattern of microneedles or via specific needles of the microneedle array, and the quality and quantity of the sedative and/or the contrast agent can be determined by a medical imaging system imaging the microneedle array and/or the released sedative and contrast agent.

The sedative can also be released transdermally to the subject by iontophoresis. Iontophoresis can use an electric charge of the sedative to accelerate or move the sedative through a least the outer layer of the skin of the subject. Iontophoresis is a method whereby a small electric current is applied between two electrodes attached with a spacing of a few centimeters to the skin. On one electrode a preferably ionized sedative is provided, for example in the form of a gel, whilst on the other electrode a source of counterions is provided. The current causes both sedative and counter ion to flow through the epidermis and dermis, where a certain amount of the drug can be transported into the blood vessels. The advantage of this approach is that the dosage can be essentially controlled by controlling the current, whereby a continuous adaptation of the sedative administration can be performed.

Further, the sedative can be applied to the subject with a high velocity of the sedative relative to the skin, such that the velocity of the sedative can be used to overcome at least the outer layer of the skin. For releasing the sedative with a relative movement with respect to the surface of the skin, for example a nozzle or a printing head can be used, which can be similar to the printing head of an inkjet printer. Since a high-speed j et pump can be connected to the outlet end of a tubular reservoir, only a small amount of the drug can be delivered to the patient's body per injection and the speed of the fluid injection can be reduced without rupturing patient's skin. Thus, the mobility and the comfort of the patient can be enhanced.

In an embodiment of the invention, the system is further configured for applying a drug penetration enhancement method, thereby improving the uptake of the transdermally released sedative by the subject.

Penetration enhancement methods can be used to improve the transdermal delivery of the sedative through at least the outer layers of the skin.

Penetration enhancers can be alcohols, sulphoxides, azote, etc. They may be combined with gels including semisolid vehicles such as micro-emulsion gels as penetration enhancers.

The local application of ultrasound with ultrasound devices can provide a thermal effect in the skin and thus loosen the barrier by increasing the gaps between elements of the skin, which can result in an improved delivery of the transdermally released sedatives through the skin during an imaging procedure.

Further, radiofrequency ablation can be used as penetration enhancement method, which uses the placement of a thin needle like an electrode or a surface antenna directly on the skin. A high frequency alternating current can be applied producing microscopic pathways in the stratum corneum of the skin. This transdermal delivery method is able to deliver a wide range of drugs and macromolecules. Local electrodes or antennas can be produced with gel like drugs as disposable devices.

A further method is mechanical rubbing or tape stripping to remove the stratum corneum layer to provide an access for transdermal delivery. A local mechanical device like a rotating surface in combination with sedation drug delivery can be realized. Parameters such as skin parameters, pH value, transepidermal water loss, or force of removal can be monitored by sensors like a camera, LED/UV light transceivers, and/or other monitoring devices. Such a device can be used in addition or as an alternative to a radiofrequency or ultrasonic ablation system.

A further method can be realized by a local resonant magnetic resonance imaging circuit, which couples to the radiofrequency field of a magnetic resonance imaging scanner in a controlled way and produces locally a heating effect. Such a device can be realized as a disposable design including a sedation transdermal gel with a penetration enhancer.

A further method can be realized by using a conductive gel with a penetration enhancer and a sedative drug and by generate heating via eddy current heating using a small local gradient coil, which may be partly similar to low frequency radiofrequency ablation.

In an embodiment of the invention, the system is further configured for calculating the amount of the sedative by classifying the subject into a category and adjusting the amount of the calculated sedative according to the category.

The measurement and control of the delivery of a sedative may lead to some kind of standard recipes which can be linked to certain people groups. The recipe information can be double-checked with former patient data from a patient record or with other previously measured parameters like weight or size of the patient, in order to get a reliable process with low patient risk. At the beginning of a procedure, the patient can be either grouped to a known recipe, or, in case of an unknown performance, an initial step by step process can be performed in order to "never overdose" the sedation. Dose and reaction behavior of the subject can be measured to estimate the dose of a next step and to double-check the outcome with an expected outcome. Thus, a reliable and patient safe procedure can be insured for every person. The documentation of this procedure using matching recipes or new low dose step by step procedures can help to fulfill upcoming local regulations. The definition and usage of standard recipes also helps to fulfill quality procedures and with the known dose also the estimated maximum movement of the patient and the movement artifacts can be estimated. This information again helps for quality control also of the image-post-processing to check for consistency and to find deviating events.

The system for controlling transdermal and automatic release of a sedative can be used in a medical imaging system like computed tomography, magnetic resonance imaging, positron emission tomography, ultrasonics, etc. during imaging of a subject. The purpose of the sedation is to achieve a good imaging quality, as movements of the patient during the imaging procedure can be reduced. The sedation procedure can be specifically adapted to a patient, for example, if a patient needs to undergo repeated imaging prodcedures. The amount of sedative and the time of relasing the sedative can be determined taking data of a previous sedation in a previous imaging procedure into account and thereby resulting in a patient specific sedation.

In an embodiment of the invention, the system further comprises a sedation level monitoring system.

The sedation level monitoring system can be fully integrated in the system of the present invention. Alternatively, it may comprise a separated unit, which can be connected to the subject and can be communicationally connected to the system of the present invention. This sedating level monitoring system measures the degree of sedation or tranquilization of the subject.

According to another aspect of the invention, there is provided a method for controlling transdermal and automatic release of a sedative during imaging of a subject. The method comprising the steps of detecting a sedation level of the subject, and calculating an amount of the sedative to be released transdermally to the subject. The method comprises further the steps of calculating a time for releasing the amount of the sedative transdermally to the subject, and releasing the calculated amount of the sedative at the calculated time transdermally to the subject.

The method for controlling transdermal and automatic release of a sedative during imaging of a subject comprises four steps. In a first step, a sedation level of a subject like a patient is detected, for example with a sedation level monitoring device. In the second step, an amount of a sedative to be released to the subject is calculated. In the third step, a time for releasing the amount of the sedative the subject is calculated. These calculations can be performed, for example, by an artificial intelligence module. In the fourth step, the calculated amount of the sedative is released at the calculated time transdermally to the subject. The sedative can be transmitted through at least the outer layer of the skin, such that the sedative can enter the blood circuit of the subject.

In an embodiment of the invention, the steps of detecting a sedation level of the subject, calculating an amount of the sedative to be released transdermally to the subject, calculating a time for releasing the amount of the sedative transdermally to the subject, and releasing the calculated amount of the sedative at the calculated time transdermally to the subject are performed by an automated system.

In this embodiment of the invention, the method for controlling transdermal and automatic release of a sedative during imaging of a subject is performed by an automated system. Thus, there is no need for a human operator of the system to perform steps or to operate the system after initializing the method. During the imaging procedure, the method can be performed by the system in order to maintain a sedation level of the subject, which may be predetermined.

In an embodiment of the invention, the step of calculating the amount of the sedative comprises comparing the detected sedation level with a set value of the sedation level.

In an embodiment of the invention, the step of calculating the amount of the sedative comprises considering variables like a type of the sedative, data of the subject, and/or the purpose of sedation.

In an embodiment of the invention, the step of calculating the amount of the sedative comprises machine learning methods.

In an embodiment of the invention, the steps of the method are carried out repeatedly during imaging of the subject.

In an embodiment of the invention, the step of calculating a time for releasing the amount of the sedative comprises determining a start time, a stop time, and a duration of releasing the sedative transdermally to the subject.

In an embodiment of the invention, the method further comprises the step of monitoring the amount of the sedative released transdermally to the subject.

In an embodiment of the invention, the step of monitoring the amount of the sedative comprises the detection of a contrast agent released together with the sedative.

In an embodiment of the invention, the step of releasing the amount of the sedative comprises one of the group consisting of: the use of a microneedle array, iontophoresis, and high velocity jetting of the sedative.

In an embodiment of the invention, the step of releasing the amount of the sedative comprises the use of a drug penetration enhancement method.

In an embodiment of the invention, the step of calculating an amount of the sedative comprises classifying the subject into a category and adjusting the amount of the calculated sedative according to the category.

According to another aspect of the invention, there is provided a computer program element, which, when executed on a processing unit, instructs the processing unit to perform the method according to any of the previous embodiments of the invention.

A computer program element can be used for instructing a processing unit to perform the steps of the method as described above.

According to another aspect of the invention, there is provided a processing unit configured for executing the computer program element according to the previous aspect of the invention.

A processing unit can be configured to execute the computer program element in order to execute the steps of the method as described above. The processing unit can be a part of a system for controlling transdermal and automatic release of a sedative during imaging of a subject.

Thus, the benefits provided by any of the above aspects equally apply to all of the other aspects and vice versa.
In a gist, the invention relates to a system and method for controlling transdermal and automatic release of a sedative during imaging of a subject. A sedation level of a patient is detected during an imaging procedure. An amount of sedative to be applied to the patient to sustain a certain level of sedation is calculated. Further, a time for releasing the amount of the sedative is calculated. The calculated amount of the sedative is released at the calculated time transdermally to the patient.
The above aspects and embodiments will become apparent from and be elucidated with reference to the exemplary embodiments described hereinafter. Exemplary embodiments of the invention will be described in the following with reference to the following drawings:

### BRIEF DESCRIPTION OF THE DRAWINGS

Fig. 1 shows a schematic set-up of a system for controlling transdermal and automatic release of a sedative during imaging of a subject according to the invention.
Fig. 2 shows a schematic set-up of an exemplary embodiment of a system for controlling transdermal and automatic release of a sedative during imaging of a subject.
Fig. 3 shows a block diagram of a method for controlling transdermal and automatic release of a sedative during imaging of a subject according to the invention.
Fig. 4 shows a block diagram of an exemplary embodiment of a method for controlling transdermal and automatic release of a sedative during imaging of a subject.
Fig. 5 shows a block diagram of a method for training an artificial intelligence module for controlling transdermal and automatic release of a sedative during imaging of a subj ect.

### DETAILED DESCRIPTION OF EMBODIMENTS

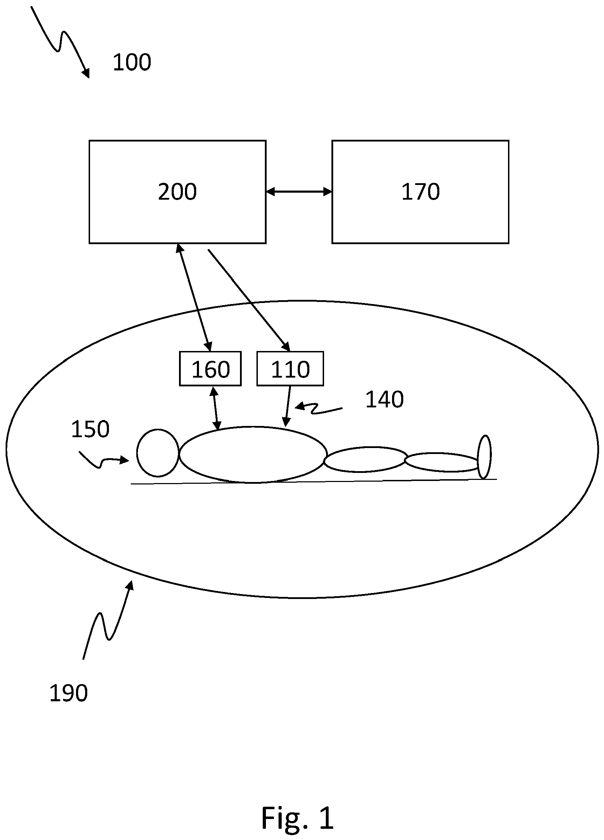

Fig. 1 shows a schematic set-up of a system 100 for controlling transdermal and automatic release of a sedative 140 during imaging of a subject 150 according to the invention. A subject 150 is located inside a medical imaging device 190. A sedation level monitoring system 160 detects and determines a sedation level of the subject 150. A processing unit 200 is communicationally connected to the sedation level monitoring system 160 and receives data regarding the sedation level. By comparing the sedation level with a set value of the sedation level, an amount of sedative 140 to be released to the subject 150 is calculated. In addition, a time is calculated, where the amount of sedative 140 is to be released to the subject 150. These calculations can be performed by the processing unit 200, or by an artificial intelligence module 170 connected communicationally to the processing unit 200. The amount of sedative 140 is released at the determined time by, for example, a microneedle array 110 to the subject. This release is performed with a system and/or a method, which releases the sedative 140 transdermally to the subject 150. The transdermal release can be a noninvasive way to incorporate the sedative 140 into the body of the subject 150. The sedative is up-taken by the subject through the skin of the subject without necessarily resulting in a violation of deeper layers of the skin.

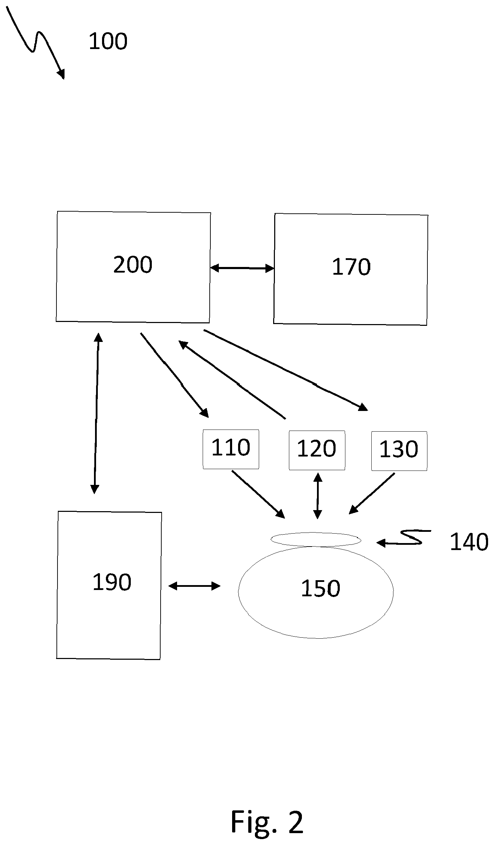

Fig. 2 shows a schematic set-up of an exemplary embodiment of a system 100 for controlling transdermal and automatic release of a sedative 140 during imaging of a subject 150. A subject 150 is located in an imaging system 190. A sedative 140 is applied to the skin of the subject 150. The system 100 comprises a microneedle array 110, which releases the sedative 140 to transdermally to the subject 150. A penetration enhancement system 130 facilitates the transdermal up-take and incorporation of the sedative 140 through the skin of the subject 150. The release of the sedative 140 as well as the up-take of the sedative 140 can be monitored. Therefore, a sensor or a camera 120 provides information to the processing unit 120, which is indicative of the amount of sedative released to and/or incorporated by the subject. Additionally or as an alternative, a contrast agent can be released in combination with the sedative 140. Thus, the medical imaging device 190 can be used to determine the amount of the contrast agent and thereby the amount of sedative 140 incorporated by the subject. Also another medical imaging device different from the medical imaging device 190 can be used for monitoring the up-take of the contrast agent. By monitoring the release and/or the up-take of the sedative 140 and/or the contrast agent, the processing unit 200 can be enabled to adjust the amount of sedative in order to maintain the required set value of the sedation level.

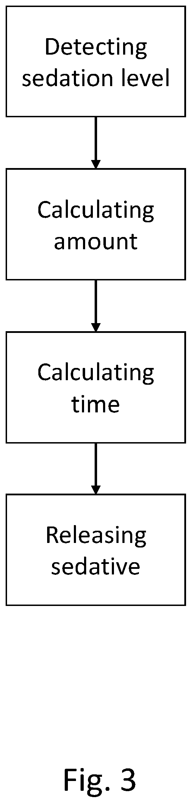

Fig. 3 shows a block diagram of a method for controlling transdermal and automatic release of a sedative 140 during imaging of a subject 150 according to the invention. The method comprise the first step of detecting a sedation level of the subject 150. This step is followed by the second step of calculating an amount of the sedative 140 to be released transdermally to the subject 150. The third step comprises calculating a time for releasing the amount of the sedative 140 transdermally to the subject 150. The fourth step comprises releasing the calculated amount of the sedative 140 at the calculated time transdermally to the subject 150.

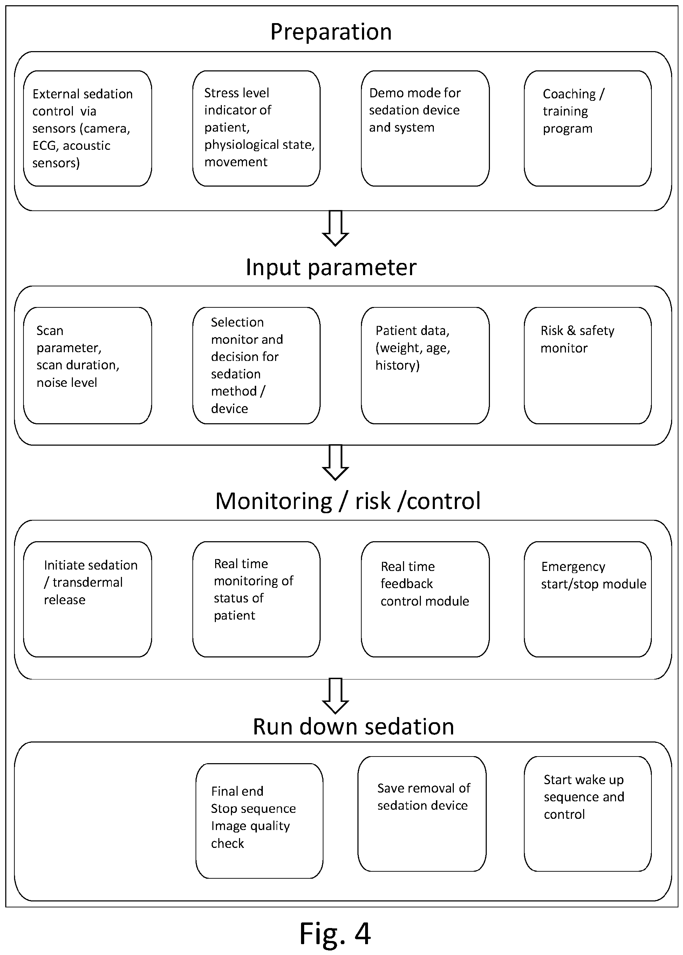

Fig. 4 shows a block diagram of an exemplary embodiment of a method for controlling transdermal and automatic release of a sedative 140 during imaging of a subject 150. In this workflow of a clinical procedure, a patient needs to undergo an autonomous scan using controlled transdermal release of a sedative. The different steps of the method can be performed on processing units, which can be located at different locations and which can be digitally linked via cable or a wireless network. For preparation of sedation, a stress level of the patient can be detected via a physiological state and movement by sensors of a sedation level monitoring device. An operator of the system can be trained by a specific training program to operate the system correctly. Further, a demo mode can be provided to ease an adaption of the patient to the system. After preparation, input parameters are transmitted to the system, which may comprise scan duration and noise level, and patient data like weight, age, or patient history. The method of sedation and a sedation device can be selected, as well as a sedation level monitoring system. Further, safety and risk of the patient can be monitored. During sedation and imaging of the patient, the sedative is released transdermally to the patient. The status of the patient can be monitored in real time, and feedback of the sedation level can be provided in order to adjust the amount of sedative transmitted to the patient. Further, an emergency stop module can be provided, which stops the procedure of sedation in case of panic of the patient or other risky occurrences. After finishing of the imaging procedure, the release of the sedative is stopped and the imaging quality can be checked. The sedation device can be removed from the patient, and a wake up sequence can be initiated.

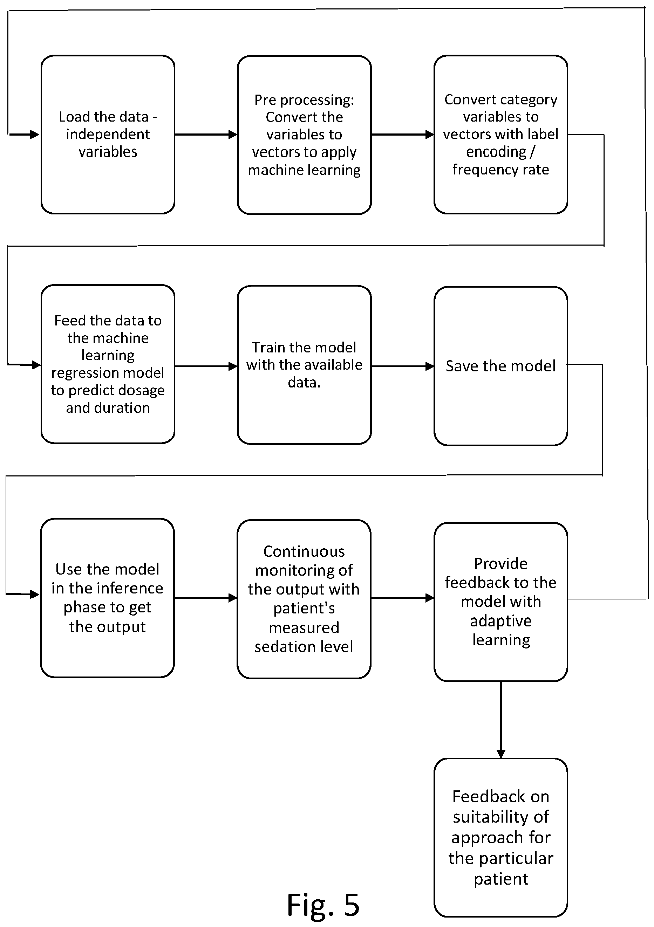

Fig. 5 shows a block diagram of a method for training an artificial intelligence module 170 for controlling transdermal and automatic release of a sedative 140 during imaging of a subject 150. The method comprises the steps of loading the data-independent variables, converting the variables to vectors to apply machine learning, converting category variables to vectors with label encoding / frequency rate, feeding the data to the machine learning regre4ssion model to predict dosage and duration, training the model with the available data, saving the model, using the model in the inference phase to get the output, monitoring continuously the output with patients measured sedation level, and providing feedback to the model with adaptive learning. Further, feedback on suitability of the approach for the particular patient can be provided.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: system
- 110: microneedle array
- 120: sensor/camera
- 130: penetration enhancement system
- 140: sedative
- 150: subject
- 160: sedation level monitoring system
- 170: artificial intelligence module
- 190: imaging device
- 200: processing unit

## Claims

1. A system (100) for controlling transdermal and automatic release of a sedative (140) during imaging of a subject (150), wherein the system (100) is configured for
detecting a sedation level of the subject (150);
calculating an amount of the sedative (140) to be released transdermally to the subject (150);
calculating a time for releasing the amount of the sedative (140) transdermally to the subject (150); and
releasing the calculated amount of the sedative (140) at the calculated time transdermally to the subject (150).

2. System (100) according to claim 1,
wherein the system (100) is further configured for calculating the amount of the sedative (140) by comparing the detected sedation level with a set value of the sedation level.

3. System (100) according to claim 2,
wherein the system (100) is further configured for calculating the amount of the sedative (140) by considering variables like a type of the sedative (140), data of the subject (150), a type of an imaging procedure, and/or the purpose of sedation.

4. System according to any of the preceding claims,
wherein the system comprises an artificial intelligence module (170) configured for calculating the amount of the sedative (140) by machine learning methods.

5. System (100) according to any of the preceding claims,
wherein the system is configured for detecting the sedation level, calculating the amount of the sedative (140), calculating the time for releasing the amount of the sedative (140), and releasing the calculated amount of the sedative (140) at the calculated time transdermally to the subject (150) repeatedly during imaging of the subject (150).

6. System (100) according to any of the preceding claims,
wherein calculating a time for releasing the amount of the sedative (140) comprises determining a start time, a stop time, and a duration of releasing the sedative (140) transdermally to the subject (150).

7. System (100) according to any of the preceding claims,
wherein the system is further configured for monitoring the amount of the sedative (140) released transdermally to the subject (150).

8. System (100) according to claim 7,
wherein the system is further configured for detecting a contrast agent released together with the sedative (140), thereby monitoring the amount of the sedative (140) released transdermally to the subject (150).

9. System (100) according to any of the preceding claims,
wherein the system is further configured for releasing the amount of the sedative (140) with one of the group consisting of: a microneedle array (110), iontophoresis, and high velocity jetting of the sedative (140).

10. System (100) according to any of the preceding claims,
wherein the system is further configured for applying a drug penetration enhancement method, thereby improving the uptake of the transdermally released sedative (140) by the subject (150).

11. System (100) according to any of the preceding claims,
wherein the system is further configured for calculating the amount of the sedative (140) by classifying the subject (150) into a category and adjusting the amount of the calculated sedative (140) according to the category.

12. A method for controlling transdermal and automatic release of a sedative (140) during imaging of a subject (150), the method comprising the steps of:
- detecting a sedation level of the subject (150);
- calculating an amount of the sedative (140) to be released transdermally to the subject (150);
- calculating a time for releasing the amount of the sedative (140) transdermally to the subject (150); and
- releasing the calculated amount of the sedative (140) at the calculated time transdermally to the subject (150).

13. Method according to claim 12,
wherein the steps of
- detecting a sedation level of the subject (150);
- calculating an amount of the sedative (140) to be released transdermally to the subject (150);
- calculating a time for releasing the amount of the sedative (140) transdermally to the subject (150); and
- releasing the calculated amount of the sedative (140) at the calculated time transdermally to the subject (150)
are performed by an automated system (100).

14. A computer program element, which, when executed on a processing unit (200), instructs the processing unit to perform the method according to any of claims 12 or 13.

15. A processing unit (200) configured for executing the computer program element according to claim 14.
